# EUROPEAN PATENT APPLICATION

(11) **EP 4 272 756 A1**
(43) Date of publication of application: **08.11.2023**
(21) Application number: 22749197.4
(22) Date of filing: 30.01.2022
(51) Int. Cl.: A61K 39/395

(54) **ANTI-IL-5 ANTIBODY FORMULATION, PREPARATION METHOD THEREFOR AND USE THEREOF**

(30) Priority: 05.02.2021 WO PCT/CN2021/075561
(71) Applicant: Bio-Thera Solutions, Ltd., Guangzhou, Guangdong 510530 (CN)
(72) Inventor: YANG, Yili, Guangzhou, Guangdong 510530 (CN); JIANG, Xuemin, Guangzhou, Guangdong 510530 (CN); LIU, Cuihua, Guangzhou, Guangdong 510530 (CN)
(74) Representative: Engelhard, Markus
(86) International application number: PCT/CN2022/075147
(87) International publication number: WO 2022/166918

(57) **Abstract**

Provided is an anti-IL-5 antibody formulation comprising an anti-IL-5 antibody, a His buffer, a stabilizer and Tween 80 and having a pH value of 5.70-6.38. The anti-IL-5 antibody formulation of the present invention exhibits better stability than the control under conditions of high temperatures, freezing-thawing, light, etc. being more suitable for clinical use.

## Description

### TECHNICAL FIELD

The present invention belongs to the field of biological formulations, and relates to an anti-IL-5 (interleukin 5) antibody formulation.

### BACKGROUND

IL-5 plays an important role in many diseases and is a common therapeutic target. Anti-IL-5 antibodies, such as mepolizumab, are capable of specifically binding to IL-5, blocking the binding of IL-5 to its receptor, and are useful in the treatment of a variety of diseases. Although mepolizumab has demonstrated its excellent efficacy, the art still requires more stable formulations of anti-IL-5 antibodies for the sake of efficacy.

### SUMMARY

Based on this, the present invention provides a stable anti-IL-5 antibody formulation.

In a first aspect, the anti-IL-5 antibody formulation of the present invention comprises an anti-IL-5 antibody and a histidine buffer.

In one embodiment, the antibody formulation has a pH value of 5.3-6.8. In one embodiment, the antibody formulation has a pH value of 5.6-6.5. In one embodiment, the antibody formulation has a pH value of 5.70-6.38. In one embodiment, the antibody formulation has a pH value of 5.7-6.3. In one embodiment, the antibody formulation has a pH value of about 5.3, about 5.6, about 5.7, about 5.8, about 5.9, about 6.0, about 6.1, about 6.2, about 6.3, about 6.4, about 6.5, about 6.8, or a range between any two of these values (inclusive) or any value therein. In one embodiment, the pH value is about 5.9 or 6.0.

In one embodiment, the histidine buffer is at a concentration of 10-30 mM. In one embodiment, the histidine buffer is at a concentration of 19-23 mM. In one embodiment, the histidine buffer is at a concentration of about 10 mM, about 12 mM, about 15 mM, about 16 mM, about 18 mM, about 19 mM, about 20 mM, about 21 mM, about 22 mM, about 23 mM, about 25 mM, about 27 mM, about 30 mM, or a range between any two of these values (inclusive) or any value therein. In one embodiment, the histidine buffer is at a concentration of 20 mM.

In one embodiment, the histidine buffer comprises histidine and histidine hydrochloride.

In one embodiment, the antibody formulation does not comprise phosphoric acid or a salt thereof. In one embodiment, the antibody formulation does not comprise citric acid or a salt thereof.

In one embodiment, the antibody formulation further comprises a stabilizer and/or a surfactant.

In one embodiment, the stabilizer is at a concentration of 35-130 mg/mL. In one embodiment, the stabilizer is sucrose or sorbitol. In one embodiment, the sucrose is at a concentration of 75-130 mg/mL. In one embodiment, the sucrose is at a concentration of 80-120 mg/mL. In one embodiment, the sucrose is at a concentration of 90-100 mg/mL. In one embodiment, the sucrose is at a concentration of about 75 mg/mL, about 78 mg/mL, about 79 mg/mL, about 80 mg/mL, about 81 mg/mL, about 82 mg/mL, about 83 mg/mL, about 88 mg/mL, about 99 mg/mL, about 105 mg/mL, about 110 mg/mL, about 117 mg/mL, about 118 mg/mL, about 119 mg/mL, about 120 mg/mL, about 121 mg/mL, about 122 mg/mL, about 123 mg/mL, about 130 mg/mL, or a range between any two of these values (inclusive) or any value therein.

In one embodiment, the sorbitol is at a concentration of 35-50 mg/mL. In one embodiment, the sorbitol is at a concentration of 38-42 mg/mL. In one embodiment, the sorbitol is at a concentration of about 35 mg/mL, about 38 mg/mL, about 39 mg/mL, about 40 mg/mL, about 41 mg/mL, about 42 mg/mL, about 45 mg/mL, about 50 mg/mL, or a range between any two of these values (inclusive) or any value therein.

In one embodiment, the surfactant is Tween 80 (i.e., polysorbate 80) or Tween 20 (i.e., polysorbate 20). In one embodiment, the surfactant is at a concentration of 0.1-0.4 mg/mL. In one embodiment, the surfactant is at a concentration of about 0.1 mg/mL, 0.15 mg/mL, 0.19 mg/mL, 0.2 mg/mL, 0.22 mg/mL, 0.24 mg/mL, 0.3 mg/mL, 0.34 mg/mL, 0.4 mg/mL, or a range between any two of these values (inclusive) or any value therein. In one embodiment, the Tween 80 is at a concentration of 0.1-0.3 mg/mL. In one embodiment, the Tween 80 is at a concentration of about 0.2 mg/mL.

In one embodiment, the formulation may further comprise a chelating agent. In one embodiment, the chelating agent is at a concentration of 0.01-0.03 mg/mL. In one embodiment, the chelating agent is at a concentration of about 0.01 mg/mL, about 0.015 mg/mL, about 0.016 mg/mL, about 0.017 mg/mL, about 0.018 mg/mL, about 0.019 mg/mL, about 0.02 mg/mL, about 0.021 mg/mL, about 0.022 mg/mL, about 0.023 mg/mL, about 0.026 mg/mL, about 0.03 mg/mL, or a range between any two of these values (inclusive) or any value therein. In one embodiment, the chelating agent is EDTA (i.e., ethylenediamine tetraacetic acid) or a salt or a hydrate thereof, or a crystal thereof, such as EDTA-2Na, disodium EDTA dihydrate. In one embodiment, disodium EDTA (i.e., EDTA-2Na) or disodium EDTA dihydrate (i.e., EDTA-2Na·2H₂O) is at a concentration of about 0.017-0.025 mg/mL. In one embodiment, EDTA-2Na is at a concentration of about 0.017 mg/mL, about 0.018 mg/mL, about 0.019 mg/mL, about 0.02 mg/mL, about 0.021 mg/mL, about 0.022 mg/mL, about 0.023 mg/mL, or about 0.025 mg/mL. In one embodiment, disodium EDTA dihydrate is at a concentration of about 0.017 mg/mL, about 0.018 mg/mL, about 0.019 mg/mL, about 0.02 mg/mL, about 0.021 mg/mL, about 0.022 mg/mL, about 0.023 mg/mL, or about 0.025 mg/mL. In one embodiment, EDTA-2Na or EDTA-2Na·2H₂O is at a molar concentration of 0.026-0.089 mM. In one embodiment, EDTA-2Na or EDTA-2Na·2H₂O is at a molar concentration of 0.026-0.080 mM. In one embodiment, EDTA-2Na or EDTA-2Na·2H₂O is at a molar concentration of about 0.026 mM, about 0.030 mM, about 0.035 mM, about 0.041 mM, about 0.046 mM, about 0.049 mM, about 0.050 mM, about 0.051 mM, about 0.052 mM, about 0.053 mM, about 0.054 mM, about 0.063 mM, about 0.070 mM, about 0.080 mM, about 0.089 mM, or a range between any two of these values (inclusive) or any value therein.

In one embodiment, the anti-IL-5 antibody in the formulation is at a concentration of 50-150 mg/mL. In one embodiment, the anti-IL-5 antibody in the formulation is at a concentration of 80-120 mg/mL. In one embodiment, the anti-IL-5 antibody in the formulation is at a concentration of about 50 mg/mL, about 60 mg/mL, about 70 mg/mL, about 80 mg/mL, about 90 mg/mL, about 94 mg/mL, about 96 mg/mL, about 98 mg/mL, about 99 mg/mL, about 100 mg/mL, about 101 mg/mL, about 102 mg/mL, about 103 mg/mL, about 104 mg/mL, about 110 mg/mL, about 120 mg/mL, about 130 mg/mL, about 140 mg/mL, about 150 mg/mL, or a range between any two of these values (inclusive) or any value therein.

In one embodiment, the antibody formulation comprises 50-150 mg/mL anti-IL-5 antibody, 10-30 mM histidine buffer, 80-120 mg/mL sucrose, and 0.1-0.4 mg/mL Tween 80. The antibody formulation has a pH value of 5.6-6.5. In one embodiment, the antibody formulation comprises 50-150 mg/mL anti-IL-5 antibody, 10-30 mM histidine buffer, 80-120 mg/mL sucrose, and 0.1-0.4 mg/mL Tween 80. The antibody formulation has a pH value of 5.70-6.38. In one embodiment, the antibody formulation comprises 50-150 mg/mL anti-IL-5 antibody, 10-30 mM histidine buffer, 35-50 mg/mL sorbitol, and 0.1-0.4 mg/mL Tween 80. The antibody formulation has a pH value of 5.70-6.38. In one embodiment, the antibody formulation has a pH value of 6.0. In one embodiment, the antibody formulation comprises 50-150 mg/mL anti-IL-5 antibody, 10-30 mM histidine buffer, 80-120 mg/mL sucrose, 0.1-0.4 mg/mL Tween 80, and 0.01-0.03 mg/mL EDTA-2Na (or 0.030-0.089 mM EDTA-2Na). The antibody formulation has a pH value of 5.6-6.5. In one embodiment, the antibody formulation comprises 50-150 mg/mL anti-IL-5 antibody, 10-30 mM histidine buffer, 80-120 mg/mL sucrose, 0.1-0.4 mg/mL Tween 80, and 0.01-0.03 mg/mL EDTA-2Na (or 0.030-0.089 mM EDTA-2Na). The antibody formulation has a pH value of 5.70-6.38. In some embodiments, the formulation comprises about 0.017 mg/mL EDTA-2Na (i.e., about 0.051 mM EDTA-2Na). In one embodiment, the antibody formulation has a pH value of 5.8-6.1.

In one embodiment, the formulation comprises about 100 mg/mL anti-IL-5 antibody, about 20 mM histidine buffer, about 120 mg/mL sucrose, about 0.2 mg/mL Tween 80, and about 0.017mg/mL EDTA-2Na. The formulation has a pH value of about 6.0.

In one embodiment, the formulation comprises about 100 mg/mL anti-IL-5 antibody, about 20 mM histidine buffer, about 80 mg/mL sucrose, about 0.2 mg/mL Tween 80, and about 0.017mg/mL EDTA-2Na. The formulation has a pH value of about 6.0.

In one embodiment, the formulation comprises about 100 mg/mL anti-IL-5 antibody, about 20 mM histidine buffer, about 80 mg/mL sucrose, and about 0.2 mg/mL Tween 80. The formulation has a pH value of about 5.7-6.4.

In one embodiment, the formulation comprises about 100 mg/mL anti-IL-5 antibody, about 20 mM histidine buffer, about 80 mg/mL sucrose, and about 0.2 mg/mL Tween 80. The formulation has a pH value of about 5.7-6.3.

In one embodiment, the formulation comprises about 100 mg/mL anti-IL-5 antibody, about 20 mM histidine buffer, about 80 mg/mL sucrose, and about 0.2 mg/mL Tween 80. The formulation has a pH value of about 6.0.

In one embodiment, the formulation comprises about 100 mg/mL anti-IL-5 antibody, about 20 mM histidine buffer, about 80 mg/mL sucrose, and about 0.2 mg/mL Tween 80. The formulation has a pH value of about 5.7.

In one embodiment, the formulation comprises about 100 mg/mL anti-IL-5 antibody, about 20 mM histidine buffer, about 80 mg/mL sucrose, and about 0.2 mg/mL Tween 80. The formulation has a pH value of about 6.3.

In one embodiment, the formulation comprises about 100 mg/mL anti-IL-5 antibody, about 20 mM histidine buffer, about 40 mg/mL sorbitol, about 0.2 mg/mL Tween 80, and about 0.017 mg/mL EDTA-2Na. The formulation has a pH value of about 6.0.

In one embodiment, the formulation comprises about 100 mg/mL anti-IL-5 antibody, about 20 mM histidine buffer, about 40 mg/mL sorbitol, and about 0.2 mg/mL Tween 80. The formulation has a pH value of about 6.0.

In one embodiment, the formulation comprises about 100 mg/mL anti-IL-5 antibody, about 20 mM histidine buffer, about 120 mg/mL sucrose, and about 0.2 mg/mL Tween 80. The formulation has a pH value of about 6.38.

In one embodiment, the formulation comprises about 100 mg/mL anti-IL-5 antibody, about 20 mM histidine buffer, about 120 mg/mL sucrose, and about 0.2 mg/mL Tween 80. The formulation has a pH value of about 6.0.

In one embodiment, the formulation comprises about 100 mg/mL anti-IL-5 antibody, about 20 mM histidine buffer, about 120 mg/mL sucrose, and about 0.2 mg/mL Tween 80. The formulation has a pH value of about 5.7. In some embodiments, a light chain of the anti-IL-5 antibody comprises a sequence set forth in SEQ ID NO: 1, a sequence having at least 90% identity to the sequence set forth in SEQ ID NO: 1, or an amino acid sequence having one or more conservative amino acid substitutions as compared to the sequence set forth in SEQ ID NO: 1; and/or
a heavy chain of the anti-IL-5 antibody comprises a sequence set forth in SEQ ID NO: 2, a sequence having at least 90% identity to the sequence set forth in SEQ ID NO: 2, or an amino acid sequence having one or more conservative amino acid substitutions as compared to the sequence set forth in SEQ ID NO: 2. In some embodiments, the light chain of the anti-IL-5 antibody comprises a sequence set forth in SEQ ID NO: 1, and the heavy chain of the anti-IL-5 antibody comprises a sequence set forth in SEQ ID NO: 2. In some embodiments, the anti-IL-5 antibody is mepolizumab or a biosimilar thereof.

In some embodiments, the present invention provides a method for preparing the formulation, comprising: taking ingredients according to the formula, adding water for dissolving and mixing the ingredients uniformly, and adjusting the pH value to 5.6-6.5 to obtain the antibody formulation.

In some embodiments, the present invention provides a method for preparing the formulation, comprising: preparing a histidine buffer, exchanging the anti-IL-5 antibody into the histidine buffer by ultrafiltration, adding an excipient, and diluting the antibody to a specified concentration to obtain the antibody formulation.

In some embodiments, the present invention provides a method for preparing the formulation, comprising: preparing 10-30 mM histidine buffer, exchanging the anti-IL-5 antibody into 10-30 mM histidine buffer by ultrafiltration, adding an excipient, and diluting the antibody to 80-120 mg/mL to obtain the antibody formulation.

In a second aspect, the present invention provides a method for preparing the formulation, comprising: taking a specified amount of the anti-IL-5 antibody, histidine, histidine hydrochloride (or histidine hydrochloride monohydrate), sucrose or sorbitol, and Tween 80, adding water for dissolving, mixing the substances described above uniformly, and adjusting the pH value (e.g., with NaOH) to 5.6-6.5. In one embodiment, the pH value is 5.70-6.38. In one embodiment, the pH value is about 6.0.

In a third aspect, the present invention provides use of the formulation in the preparation of a product for treating a disease including, but not limited to, asthma, severe eosinophilic asthma, severe asthma, uncontrolled eosinophilic asthma, eosinophilic asthma, sub-eosinophilic asthma, chronic obstructive pulmonary disease, eosinophilic granulomatosis with polyangiitis, hypereosinophilic syndrome, nasal polyposis, bullous pemphigoid and eosinophilic esophagitis. In a fourth aspect, the present invention provides an antibody pharmaceutical product for treating an IL-5-associated disease comprising the anti-IL-5 antibody formulation described above and a container for storing the formulation. The pharmaceutical product may also comprise instructions for use. The container may be any container conventionally used in the art for storing drugs, such as pre-filled containers, pre-filled syringes, injection pens, vials (e.g., penicillin bottles), ampoules, sachets, and the like. The antibody formulation of the present invention has improved stability at high temperature and room temperature, can be kept in a liquid form, and is convenient to use.

The formulation of the present invention exhibits better stability than the control under high temperature, freeze-thaw and light conditions, and is more suitable for clinical use.

In one embodiment, the formulation of the present invention is stable when stored at 40 °C for at least 2 weeks. In one embodiment, the formulation of the present invention is stable when stored at 40 °C for at least 4 weeks. In one embodiment, the formulation of the present invention is stable when stored under a light condition for at least 14 days.

In one embodiment, the formulation of the present invention is stable after at least 5 repeated freeze-thaw cycles.

### DETAILED DESCRIPTION

The present invention will be illustrated by the following specific embodiments, but the content of the present invention is not limited thereto.

"About" or "approximately" refers to a general error range for corresponding values as readily understood by those skilled in the relevant art. In some embodiments, "about" or "approximately" mentioned herein refers to the numerical values described as well as its ranges of ± 10%, ± 5%, or ± 1%.

"Comprise" or "include" means that the compositions, methods, and the like comprise the listed elements (e.g., ingredients of the compositions, steps of the methods, and the like), but do not exclude the others. When used to define the compositions and methods, "consisting essentially of..." means excluding other elements that have a fundamental impact on the combination for its intended use, but not excluding elements that do not substantially affect the characteristics of the compositions or methods. "Consisting of..." means excluding elements not specifically listed. Embodiments defined by each of these transition terms are all within the scope of the present invention. For example, when a composition is described as including ingredients A, B, and C, a composition consisting essentially of A, B, and C, and a composition consisting of A, B, and C are independently within the scope of the present invention.

"Amino acid" refers to an organic compound containing both an amino group and a carboxyl group, such as an α-amino acid that can be encoded by a nucleic acid, either directly or in the form of a precursor. A single amino acid is encoded by a nucleic acid consisting of three nucleotides (so-called codon or base triplet). Each amino acid is encoded by at least one codon. The encoding of the same amino acid by different codons is known as "codon degeneracy". Amino acids include natural amino acids and non-natural amino acids. Natural amino acids include alanine (three-letter code: ala, one-letter code: A), arginine (arg, R), asparagine (asn, N), aspartic acid (asp, D), cysteine (cys, C), glutamine (gln, Q), glutamic acid (glu, E), glycine (gly, G), histidine (his, H), isoleucine (ile, 1), leucine (leu, L), lysine (lys, K), methionine (met, M), phenylalanine (phe, F), proline (pro, P), serine (ser, S), threonine (thr, T), tryptophan (trp, W), tyrosine (tyr, Y), and valine (val, V).

"Conservative amino acid substitution" refers to the substitution of one amino acid residue with another amino acid residue having a side chain (R group) with similar chemical properties (e.g., charge or hydrophobicity). Generally, conservative amino acid substitutions do not substantially alter the functional properties of the protein. Examples of groups of amino acids that have side chains with similar chemical properties include: 1) aliphatic side chains: glycine, alanine, valine, leucine and isoleucine; 2) aliphatic hydroxyl side chains: serine and threonine; 3) amidecontaining side chains: asparagine and glutamine; 4) aromatic side chains: phenylalanine, tyrosine and tryptophan; 5) basic side chains: lysine, arginine and histidine; and 6) acidic side chains: aspartic acid and glutamic acid.

A number of amino acids of "conservative amino acid substitutions of a heavy light or a light chain" is about 1, about 2, about 3, about 4, about 5, about 6, about 8, about 9, about 10, about 11, about 13, about 14, about 15, about 18, about 19, about 22, about 24, about 25, about 29, about 31, about 35, about 38, about 41, about 45 conservative amino acid substitutions, or a range between any two of these values (inclusive) or any value therein.

"At least 90% identity" refers to about 90% identity, about 91% identity, about 92% identity, about 93% identity, about 94% identity, about 95% identity, about 96% identity, about 97% identity, about 98% identity, about 99% identity, or a range between any two of these values (inclusive) or any value therein. "Homology", "identity" or "similarity" refers to sequence similarity between two peptides or between two nucleic acid molecules. Homology can be determined by comparing the positions that can be aligned in the sequences. When a position of the compared sequences is occupied by the same base or amino acid, then the molecules are homologous at that position. The degree of homology between the sequences is a function of the number of matching or homologous positions shared by the sequences.

The active ingredient in the formulation of the present invention is an anti-IL-5 antibody, including but not limited to monoclonal antibodies, chimeric antibodies, dAbs (domain antibodies), single-chain antibodies, Fab, Fab- and F(ab')2 fragments, Fv, scFv and Fab expression libraries. In some embodiments, the anti-IL-5 antibody is a recombinant anti-IL-5 monoclonal antibody, which is prepared by culturing cells that efficiently express the recombinant anti-IL-5 monoclonal antibody, isolating the antibody, and purifying the antibody to a high degree. In some embodiments of the present invention, the light chain of the anti-IL-5 antibody has an amino acid sequence set forth in SEQ ID NO: 1, and the heavy chain has an amino acid sequence set forth in SEQ ID NO: 2. In some embodiments, the anti-IL-5 antibody is mepolizumab, such as an antibody in Nucala^{®}, or a biosimilar thereof.

The "treatment" of a patient's disease means: (1) preventing a disease from occurring in a patient who is prone to the disease or has not shown symptoms of the disease; (2) inhibiting the disease or preventing its development; or (3) relieving the disease or making it regress.

The term "buffer" is also referred to as a buffer system, and a histidine buffer includes histidine and histidine salts such as hydrochloride. The amount of the buffer in the present invention means the total amount of the buffer pair in the buffer system constituting the buffer. In some embodiments, molar concentration is taken as a unit of the amount of the buffer, the value of which refers to the molar concentration of the buffer pair in the buffer system of the buffer. For example, where histidine buffer consisting of histidine and histidine hydrochloride is used as the buffer, a given concentration of histidine buffer (e.g., 20 mM) is the combined concentration of histidine and histidine hydrochloride.

The formulation of the present invention can be prepared with the excipients or the hydrates thereof. For example, histidine hydrochloride, also known as histidine hydrochloride, may be anhydrous histidine hydrochloride, or histidine hydrochloride hydrate, such as histidine hydrochloride monohydrate; and EDTA-2Na may be anhydrous EDTA-2Na or EDTA-2Na hydrate, such as EDTA-2Na·2H₂O. For example, "0.051 mM EDTA-2Na", may be a 1 L solution formed by dissolving 0.051 mmol EDTA-2Na or EDTA-2Na·2H₂O in a solvent; and 0.017 mg EDTA-2Na includes 0.017 mg EDTA-2Na or the corresponding amount of hydrate (e.g., 0.019 mg EDTA-2Na·2H₂O).

Tween is also known as polysorbate (e.g., Tween 20 is known as polysorbate 20, and Tween 80 is known as polysorbate 80).

"Stability" and "stable" herein mean that the antibody (including the antibody fragment thereof) does not, or only minimally, undergo aggregation, degradation, or fragmentation under given conditions of manufacture, preparation, transportation and/or storage in a liquid formulation comprising the antibody. A "stable" formulation maintains biological activity under given conditions of manufacture, preparation, transportation and/or storage. The stability of the antibody can be evaluated by the extent of aggregation, degradation or fragmentation of the formulation and the like as measured by technologies such as SEC-HPLC, IEC-HPLC, CE-SDS (NR), visual inspection and turbidity, insoluble particles, and detection of particle size by DLS.

In some embodiments, the formulation provided herein has a pH value of about 5.70-6.38. In some embodiments, the formulation provided herein has a pH value of about 6.0.

The formulation provided herein has enhanced stability. In some embodiments, the formulation of the present invention is stable after at least 5 freeze-thaw cycles. In other embodiments, the formulation of the present invention remains stable after being placed at a high temperature of 40 °C for 1 week or 2 weeks. In other embodiments, the formulation of the present invention still remains stable under a light condition of 4000 lx for a period of time.

The present invention further provides a method for treating an IL-5-associated disease, comprising administering to a patient the antibody formulation.

In the following examples, reagents and instruments used are those conventional in the art and commercially available unless otherwise specified; the methods used are conventional in the art, and those skilled in the art can undoubtedly implement the methods and obtain the corresponding results according to the content of the examples.

The anti-IL-5 antibody used in the examples below is mepolizumab. Mepolizumab comprises 2 sequence-identical light chains and 2 sequence-identical heavy chains with the amino acid sequences set forth in SEQ ID NO: 1 and SEQ ID NO: 2, respectively:
SEQ ID NO: 1 (light chain sequence): 
DIVMTQSPDSLAVSLGERATINCKSSQSLLNSGNQKNYLAWYQQKPGQPPKLLIYGASTR
ESGVPDRFSGSGSGTDFTLTISSLQAEDVAVYYCQNVHSFPFTFGGGTKLEIKRTVAAPSV
FIFPPSDEQLKSGTASVVCLLNNFYPREAKVQWKVDNALQSGNSQESVTEQDSKDSTYS
LSSTLTLSKADYEKHKVYACEVTHQGLSSPVTKSFNRGEC;
SEQ ID NO: 2 (heavy chain sequence):

QVTLRESGPALVKPTQTLTLTCTVSGFSLTSYSVHWVRQPPGKGLEWLGVIWASGGTDY
NSALMSRLSISKDTSRNQVVLTMTNMDPVDTATYYCARDPPSSLLRLDYWGRGTPVTVS
SASTKGPSVFPLAPSSKSTSGGTAALGCLVKDYFPEPVTVSWNSGALTSGVHTFPAVLQS
SGLYSLSSVVTVPSSSLGTQTYICNVNHKPSNTKVDKRVEPKSCDKTHTCPPCPAPELLG
GPSVFLFPPKPKDTLMISRTPEVTCVVVDVSHEDPEVKFNWYVDGVEVHNAKTKPREEQ
YNSTYRVVSVLTVLHQDWLNGKEYKCKVSNKALPAPIEKTISKAKGQPREPQVYTLPPS
REEMTKNQVSLTCLVKGFYPSDIAVEWESNGQPENNYKTTPPVLDSDGSFFLYSKLTVD
KSRWQQGNVFSCSVMHEALHNHYTQKSLSLSPGK.

The anti-IL-5 antibody used in the following examples was expressed by CHO cells and prepared by culturing CHO cells, isolating the antibody, and purifying the antibody to a high degree. The CHO cells, antibody expression methods, plasmid construction, purification methods, and the like were purchased and operated according to a conventional manner.

In the following examples, SEC-HPLC, IEC-HPLC and osmotic pressure detection were carried out in a conventional manner.

### Example 1: Effect of Different pH Values on Stability of Formulations

20 mM histidine buffer (3.1005 g histidine was weighed, dissolved in 1 L water, and mixed well) at pH 7.7, and 20 mM histidine salt buffer (4.2032 g histidine hydrochloride was weighed, dissolved in 1 L water, and mixed well) at pH 4.1 were prepared respectively. Then the two buffers described above were adjusted with each other to obtain 20 mM histidine buffers at pH 5.2 and pH 6.5. The purified anti-IL-5 antibodies were ultrafiltered into buffers at pH 5.2 and pH 6.5, respectively, and the two pH samples obtained were adjusted with each other. Sucrose and Tween 80 were added according to the formulation ingredients in Table 1, and then the antibodies were diluted to 100 mg/mL to obtain samples at pH 6.77, 6.38, 6.00, 5.70, and 5.34, respectively, all with an antibody concentration of 100 mg/mL.

The other ingredients and contents of each formulation were detailed in Table 1 below:

**Table 1: Formulations for different pH values**

| **Formulation No.** | **4** | **5** | **6** | **7** | **8** |
|---|---|---|---|---|---|
| His (histidine buffer) (mM) | 20 | 20 | 20 | 20 | 20 |
| Sucrose (mg/mL) | 120 | 120 | 120 | 120 | 120 |
| Tween 80 (mg/mL) | 0.2 | 0.2 | 0.2 | 0.2 | 0.2 |
| pH value | 6.77 | 6.38 | 6.00 | 5.70 | 5.34 |

### Test method:

The prepared sample aliquots were placed under a condition of 40 °C, and were sampled for detection on day 0, day 3, day 7, and day 14, respectively. The detection items were SEC-HPLC and IEC-HPLC. In this example, the effect of different pH values on the stability of formulations with the same ingredients was investigated, and the detection results were as follows:

**Table 2: SEC-HPLC data for different formulations at 40 °C**

| | 40 °C-day 0 | | | 40 °C-day 3 | | | 40 °C-day 7 | | | 40 °C-day 14 | | | Day 0-day 14 Amount of monomer decrease % |
|---|---|---|---|---|---|---|---|---|---|---|---|---|---|
| | Aggregate % | Monomer % | Fragment % | Aggregate % | Monomer % | Fragment % | Aggregate % | Monomer % | Fragment % | Aggregate % | Monomer % | Fragment % | |
| Formulation 4 | 0.77 | 98.22 | 1.01 | 1.34 | 97.47 | 1.19 | 1.28 | 97.30 | 1.41 | 1.40 | 96.83 | 1.77 | 1.39 |
| Formulation 5 | 0.76 | 98.21 | 1.03 | 106 | 97.75 | 1.19 | 1.14 | 97.45 | 1.41 | 1.22 | 97.07 | 1.71 | 1.14 |
| Formulation 6 | 0.71 | 98.25 | 1.05 | 0.62 | 98.22 | 1.16 | 1.01 | 97.56 | 1.42 | 1.10 | 97.15 | 1.75 | 1.10 |
| Formulation 7 | 0.59 | 98.39 | 1.02 | 0.67 | 98.17 | 1.16 | 0.95 | 97.62 | 1.44 | 1.01 | 97.16 | 1.84 | 123 |
| Formulation 8 | 0.64 | 98.33 | 1.03 | 0.77 | 98.00 | 1.23 | 0.87 | 97.61 | 1.51 | 0.92 | 97.11 | 1.97 | 1.22 |

As can be seen from Table 2, each formulation maintained a good monomer purity at pH 5.34-6.77, and the SEC monomer purities of formulations 5 to 8 (pH 5.34-pH 6.38) decreased less than that of formulation 4 (pH 6.77).

**Table 3: IEC-HPLC data for different formulations at 40 °C**

| | 40 °C-day 0 | | | 40 °C-day 3 | | | 40 °C-day 7 | | | 40 °C-day 14 | | | Day 0-day 14 (Main Peak decrease) % |
|---|---|---|---|---|---|---|---|---|---|---|---|---|---|
| | Acidic peak % | Main peak % | Basic peak% | Acidic peak % | Main peak % | Basic peak% | Acidic peak % | Main peak % | Basic peak % | Acidic peak % | Main peak % | Basic peak % | |
| Formulation 4 | 34.83 | 57.01 | 8.16 | 39.23 | 52.78 | 7.99 | 44.85 | 47.66 | 7.49 | 53.04 | 40.15 | 6.81 | 16.86 |
| Formulation 5 | 34.90 | 57.01 | 8.09 | 38.06 | 53.63 | 8.31 | 42.28 | 49.65 | 8.07 | 48.92 | 43.43 | 7.65 | 13.58 |
| Formulation 6 | 34.58 | 57.13 | 8.28 | 37.57 | 53.59 | 8.84 | 41.15 | 49.88 | 8.97 | 47.03 | 44.01 | 8.97 | 13.12 |
| Formulation 7 | 34.54 | 57.41 | 8.05 | 37.51 | 53.56 | 8.93 | 41.53 | 48.98 | 9.49 | 47.60 | 42.57 | 9.83 | 14.84 |
| Formulation 8 | 34.58 | 57.10 | 8.32 | 38.77 | 51.88 | 9.35 | 43.54 | 46.40 | 10.06 | 50.78 | 38.72 | 10.50 | 18.38 |

As can be seen from the results in Table 3, each formulation maintained a good main peak content at pH 5.34-6.77, and the IEC main peak contents of formulations 5 to 7 (pH 5.70-pH 6.38) decreased more slowly than that of formulations 8 (pH 5.34) and 4 (pH 6.77).

It can be seen from the results described above that each formulation had good stability at pH 5.70-pH 6.38 with the same ingredients.

### Example 2: Effect of Different Buffers on Stability of Formulations

The effect of different buffers on the stability of formulations was analyzed. Control formulation 3 (ingredients: 100 mg/mL anti-IL-5 antibody, 4.5 mM citric acid monohydrate, 15.5 mM disodium hydrogen phosphate heptahydrate, 120 mg/mL sucrose, and 0.2 mg/mL Tween 80, pH 6.36) and formulation 5 differed only in the buffer, and the other ingredients, contents and pH values were substantially the same. The two formulations were compared and the results were as follows:

**Table 4: SEC-HPLC data under different buffer conditions**

| | 40 °C-day 0 | | | 40 °C-day 3 | | | 40 °C-day 7 | | | 40 °C-day 14 | | | Day 0-day 14 (Monomer decrease) % |
|---|---|---|---|---|---|---|---|---|---|---|---|---|---|
| | Aggregate % | Monomer % | Fragment % | Aggregate % | Monomer % | Fragment % | Aggregate % | Monomer % | Fragment % | Aggregate % | Monomer % | Fragment % | |
| Formulation 3 | 1.13 | 97.86 | 1.01 | 1.27 | 97.60 | 1.14 | 1.63 | 96.94 | 1.43 | 1.77 | 96.49 | 1.75 | 1.37 |
| Formulation 5 | 0.76 | 98.21 | 1.03 | 106 | 97.75 | 1.19 | 1.14 | 97.45 | 1.41 | 1.22 | 97.07 | 1.71 | 1.14 |

As can be seen from Table 4, the SEC-HPLC monomer content of formulation 5 decreased more slowly than that of formulation 3, indicating that the buffer of formulation 5 was more capable of keeping the formulation stable.

**Table 5: IEC-HPLC data of different buffers at 40 °C**

| | 40 °C-day 0 | | | 40 °C-day 3 | | | 40 °C-day 7 | | | 40 °C-day 14 | | | Day 0-day 14 (Main peak decrease) % |
|---|---|---|---|---|---|---|---|---|---|---|---|---|---|
| | Acidic peak % | Main peak % | Basic peak% | Acidic peak % | Main peak % | Basic peak% | Acidic peak % | Main peak % | Basic peak % | Acidic peak % | Main peak % | Basic peak % | |
| Formulation 3 | 34.76 | 56.81 | 8.43 | 38.58 | 52.94 | 8.49 | 43.21 | 48.71 | 8.07 | 50.68 | 41.89 | 7.42 | 14.92 |
| Formulation 5 | 34.90 | 57.01 | 8.09 | 38.06 | 53.63 | 8.31 | 42.28 | 49.65 | 8.07 | 48.92 | 43.43 | 7.65 | 13.58 |

As can be seen from Table 5, the IEC-HPLC main peak content of formulation 5 decreased more slowly than that of formulation 3. As can be seen from the data in Table 4 described above, different buffers had a significant effect on the stability of the formulation, with His buffer being better than a buffer consisting of citric acid and disodium hydrogen phosphate.

As can be seen from the above experimental results, for the anti-IL-5 antibody formulation, the formulation was more stable when the pH range was 5.70-6.38 and the buffer was His buffer.

### Example 3: Study of Formulation Stability

In this example, the stability of several formulations was investigated and compared with that of a control.

The preparation method for 20 mM His buffer (i.e., 20 mM histidine buffer) comprises the following steps: 99.216 g of histidine and 201.752 g of histidine hydrochloride were weighed, dissolved in 80 L of ultrapure water, and mixed to obtain a solution with a pH value of 5.8.The anti-IL-5 antibody was exchanged into 20 mM His buffer by ultrafiltration, the rest excipients were added according to the formulation ingredients in Table 6, and then the antibody was diluted to 100 mg/mL to obtain samples of formulations 6 and 14-18.

**Table 6: Formulation samples of different ingredients**

| **Formulation No.** | **6** | **12 (control)** | **14** | **15** | **16** | **17** | **18** |
|---|---|---|---|---|---|---|---|
| His buffer (mM) | 20 | | 20 | 20 | 20 | 20 | 20 |
| Citric acid monohydrate (mM) | | 4.5 | | | | | |
| Anhydrous disodium hydrogen phosphate (mM) | | 15.5 | | | | | |
| EDTA-2Na dihydrate (mg/mL) | | 0.019 | 0.019 | 0.019 | | 0.019 | |
| Sucrose (mg/mL) | 120 | 120 | 120 | 80 | 80 | | |
| Sorbitol (mg/mL) | | | | | | 40 | 40 |
| Tween 80 (mg/mL) | 0.2 | 0.2 | 0.2 | 0.2 | 0.2 | 0.2 | 0.2 |
| pH value | 5.95 | 6.25 | 5.97 | 5.98 | 5.95 | 5.97 | 5.98 |

### Test method:

The prepared and packaged samples were placed under conditions of 40 °C, light and repeated freeze-thaw at -60 °C, and sampled for detection after a certain period of time. The placement conditions and sampling time points were as follows in Table 7:

**Table 7: Sample placement conditions and sampling time**

| Conditions | Sampling time |
|---|---|
| 40°C | Day 0, week 1, week 2, week 3, week 4 |
| light | Day 0, day 1, day 3, day 7, day 14 |
| Repeated freeze-thaw at -60 °C | 0 time (DR0), 3 times (DR3), 5 times (DR5) |

The detection items were as follows:
detection on day 0: osmotic pressure, SEC-HPL, and IEC-HPLC;
other time points: SEC-HPLC, and IEC-HPLC (choosing some points).

### Test results

### 1. Detection results of osmotic pressure

The osmotic pressure of each formulation was measured on day 0 and the results were shown in Table 8.

**Table 8: Osmotic pressure data of each formulation sample**

| Detection item | Formulation 6 | Formulation 12 | Formulation 14 | Formulation 15 | Formulation 16 | Formulation 17 | Formulation 18 |
|---|---|---|---|---|---|---|---|
| Molar concentration of osmotic pressure (mOsmol/kg) | 492 | 489 | 514 | 324 | 329 | 301 | 277 |

As can be seen from Table 8, formulations 15-18 had osmotic pressures approaching the physiological range and were more suitable for subcutaneous injection.

### 2. Screening results at high temperature

Each sample placed at 40 °C was subjected to SEC-HPLC and IEC-HPLC detections, and the results were shown in Tables 9 and 10 respectively.

**Table 9: SEC-HPLC data of each formulation sample at high temperature (40 °C)**

| Sample | | Day 0 | Day 7 | Day 14 | Day 24 | Day 28 | Days 0-28 (Monomer decrease) |
|---|---|---|---|---|---|---|---|
| Formulation 6 | Aggregate (%) | 2.15 | 2.39 | 2.47 | 2.55 | 2.75 | 1.96 |
| | Monomer (%) | 96.95 | 96.33 | 95.95 | 95.58 | 94.99 | |
| | Fragment (%) | 0.90 | 1.28 | 1.58 | 1.87 | 2.25 | |
| Formulation 12 | Aggregate (%) | 2.32 | 2.91 | 3.03 | 3.19 | 3.46 | 2.71 |
| | Monomer (%) | 96.76 | 95.80 | 95.35 | 94.81 | 94.05 | |
| | Fragment (%) | 0.92 | 1.29 | 1.62 | 2.00 | 2.48 | |
| Formulation 14 | Aggregate (%) | 2.15 | 2.44 | 2.56 | 2.54 | 2.68 | 1.90 |
| | Monomer (%) | 96.93 | 96.25 | 95.77 | 95.55 | 95.03 | |
| | Fragment (%) | 0.91 | 1.31 | 1.67 | 1.91 | 2.29 | |
| Formulation 15 | Aggregate (%) | 2.19 | 2.47 | 2.45 | 2.57 | 2.72 | 1.91 |
| | Monomer (%) | 96.89 | 96.21 | 95.95 | 95.57 | 94.98 | |
| | Fragment (%) | 0.92 | 1.32 | 1.60 | 1.85 | 2.30 | |
| Formulation 16 | Aggregate (%) | 2.15 | 2.49 | 2.53 | 2.63 | 2.85 | 2.06 |
| | Monomer (%) | 96.97 | 96.27 | 95.90 | 95.57 | 94.91 | |
| | Fragment (%) | 0.88 | 1.24 | 1.57 | 1.80 | 2.24 | |
| Formulation 17 | Aggregate (%) | 2.12 | 2.46 | 2.71 | 2.75 | 2.85 | 2.18 |
| | Monomer (%) | 97.02 | 96.26 | 95.58 | 95.31 | 94.84 | |
| | Fragment (%) | 0.86 | 1.28 | 1.72 | 1.94 | 2.31 | |
| Formulation 18 | Aggregate (%) | 2.18 | 2.51 | 2.59 | 2.68 | 2.85 | 2.11 |
| | Monomer (%) | 96.96 | 96.19 | 95.86 | 95.50 | 94.85 | |
| | Fragment (%) | 0.86 | 1.30 | 1.55 | 1.82 | 2.30 | |

As can be seen from Table 9, the SEC-HPLC monomer purity of the control formulation 12 decreased most rapidly. The formulations 6 and 14-18 can maintain good stability, and were more stable than the control formula, especially the formulations 6, 14-16.

**Table 10: IEC-HPLC data at high temperature (40 °C)**

| Sample | | Day 0 | Day 7 | Day 14 | Day 24 | Day 28 | Days 0-28 (Main peak decrease) |
|---|---|---|---|---|---|---|---|
| Formulation 6 | Acidic peak (%) | 29.20 | 33.75 | 39.42 | 47.53 | 48.34 | 21.91 |
| | Main peak (%) | 60.88 | 53.48 | 47.69 | 40.97 | 38.97 | |
| | Basic peak (%) | 9.92 | 12.76 | 12.89 | 11.49 | 12.69 | |
| Formulation 12 | Acidic peak (%) | 29.94 | 36.53 | 43.74 | 53.99 | 56.15 | 24.57 |
| | Main peak (%) | 60.29 | 55.29 | 47.81 | 38.94 | 35.72 | |
| | Basic peak (%) | 9.76 | 8.19 | 8.44 | 7.06 | 8.14 | |
| Formulation 14 | Acidic peak (%) | 29.63 | 33.91 | 39.18 | 46.53 | 48.65 | 21.43 |
| | Main peak (%) | 60.79 | 53.43 | 47.70 | 41.48 | 39.36 | |
| | Basic peak (%) | 9.58 | 12.67 | 13.13 | 11.99 | 11.99 | |
| Formulation 15 | Acidic peak (%) | 29.60 | 33.29 | 38.31 | 46.18 | 48.22 | 21.93 |
| | Main peak (%) | 60.74 | 54.24 | 48.80 | 41.66 | 38.81 | |
| | Basic peak (%) | 9.66 | 12.48 | 12.89 | 12.16 | 12.97 | |
| Formulation 16 | Acidic peak (%) | 30.26 | 33.14 | 38.37 | 46.00 | 48.55 | 21.43 |
| | Main peak (%) | 60.12 | 54.47 | 48.82 | 41.74 | 38.69 | |
| | Basic peak (%) | 9.62 | 12.39 | 12.81 | 12.26 | 12.76 | |
| Formulation 17 | Acidic peak (%) | 29.94 | 34.00 | 39.32 | 46.48 | 47.87 | 22.54 |
| | Main peak (%) | 60.81 | 53.24 | 47.27 | 41.06 | 38.27 | |
| | Basic peak (%) | 9.25 | 12.76 | 13.41 | 12.46 | 13.86 | |
| Formulation 18 | Acidic peak (%) | 30.10 | 33.39 | 38.58 | 46.10 | 48.37 | 22.20 |
| | Main peak (%) | 60.75 | 54.03 | 48.35 | 41.23 | 38.55 | |
| | Basic peak (%) | 9.15 | 12.58 | 13.07 | 12.66 | 13.08 | |

As can be seen from the results in Table 10, the IEC main peak contents of formulations 6 and 14-18 all decreased less than that of the control formulation 12, and in particular, the IEC main peak contents of formulations 14 and 16 decreased most slowly.

### 3. Screening results under light

Each sample placed under a light condition (25 °C, 4000 ± 500 lx) was subjected to SEC-HPLC and IEC-HPLC detections, and the results were as follows:

**Table 11: SEC-HPLC data of each formulation sample under the light condition**

| Sample | | Day 0 | Day 1 | Day 3 | Day 7 | Day 14 | Days 0-14 (Monomer decrease) |
|---|---|---|---|---|---|---|---|
| Formulation 6 | Aggregate (%) | 2.03 | 2.89 | 3.26 | 4.16 | 4.39 | 2.73 |
| | Monomer (%) | 97.15 | 96.03 | 95.80 | 94.79 | 94.42 | |
| | Fragment (%) | 0.82 | 1.07 | 0.94 | 1.04 | 1.19 | |
| Formulation 12 | Aggregate (%) | 2.21 | 5.37 | 6.90 | 9.29 | 10.88 | 8.75 |
| | Monomer (%) | 96.85 | 93.80 | 92.22 | 89.84 | 88.10 | |
| | Fragment (%) | 0.94 | 0.82 | 0.88 | 0.87 | 1.02 | |
| Formulation 14 | Aggregate (%) | 2.45 | 2.92 | 3.37 | 3.51 | 4.02 | 1.75 |
| | Monomer (%) | 96.60 | 96.13 | 95.66 | 95.55 | 94.85 | |
| | Fragment (%) | 0.95 | 0.95 | 0.97 | 0.94 | 1.14 | |
| Formulation 15 | Aggregate (%) | 2.26 | 2.81 | 3.34 | 3.97 | 4.37 | 2.43 |
| | Monomer (%) | 96.86 | 96.29 | 95.73 | 95.05 | 94.43 | |
| | Fragment (%) | 0.88 | 0.90 | 0.93 | 0.98 | 1.20 | |
| Formulation 16 | Aggregate (%) | 2.12 | 2.80 | 3.43 | 4.11 | 4.06 | 2.31 |
| | Monomer (%) | 97.09 | 96.31 | 95.55 | 94.93 | 94.78 | |
| | Fragment (%) | 0.79 | 0.90 | 1.02 | 0.96 | 1.15 | |
| Formulation 17 | Aggregate (%) | 2.04 | 2.80 | 3.47 | 4.09 | 4.57 | 2.86 |
| | Monomer (%) | 97.14 | 96.32 | 95.59 | 94.90 | 94.28 | |
| | Fragment (%) | 0.81 | 0.87 | 0.94 | 1.00 | 1.15 | |
| Formulation 18 | Aggregate (%) | 2.19 | 2.85 | 3.59 | 4.31 | 4.53 | 2.44 |
| | Monomer (%) | 96.77 | 96.26 | 95.51 | 94.69 | 94.33 | |
| | Fragment (%) | 1.04 | 0.90 | 0.90 | 1.01 | 1.14 | |

As can be seen from Table 11, the SEC-HPLC monomer purity of the control formulation 12 decreased most rapidly, significantly higher than that of the other formulations.

**Table 12: IEC-HPLC data of each formulation sample under the light condition**

| Sample | | Day 0 | Day 1 | Day 3 | Day 7 | Days 0-7 (Main peak decrease %) |
|---|---|---|---|---|---|---|
| Formulation 6 | Acidic peak (%) | 27.87 | 32.02 | 35.88 | 41.32 | 22.19 |
| | Main peak (%) | 59.84 | 51.51 | 46.44 | 37.65 | |
| | Basic peak (%) | 12.29 | 16.48 | 17.67 | 21.03 | |
| Formulation 12 | Acidic peak (%) | 28.15 | 31.04 | 33.84 | 36.90 | 21.36 |
| | Main peak (%) | 59.83 | 50.14 | 45.70 | 38.47 | |
| | Basic peak (%) | 12.02 | 18.82 | 20.46 | 24.63 | |
| Formulation 14 | Acidic peak (%) | 28.06 | 32.54 | 36.49 | 37.44 | 15.95 |
| | Main peak (%) | 59.66 | 50.61 | 45.66 | 43.71 | |
| | Basic peak (%) | 12.28 | 16.85 | 17.85 | 18.86 | |
| Formulation 15 | Acidic peak (%) | 27.95 | 32.07 | 35.69 | 39.62 | 19.29 |
| | Main peak (%) | 59.95 | 51.28 | 47.08 | 40.66 | |
| | Basic peak (%) | 12.10 | 16.66 | 17.23 | 19.72 | |
| Formulation 16 | Acidic peak (%) | 28.07 | 32.19 | 36.34 | 40.65 | 20.50 |
| | Main peak (%) | 59.71 | 51.19 | 46.02 | 39.21 | |
| | Basic peak (%) | 12.22 | 16.62 | 17.64 | 20.14 | |
| Formulation 17 | Acidic peak (%) | 28.08 | 31.96 | 36.43 | 39.77 | 19.50 |
| | Main peak (%) | 59.65 | 51.34 | 46.55 | 40.15 | |
| | Basic peak (%) | 12.27 | 16.70 | 17.02 | 20.08 | |
| Formulation 18 | Acidic peak (%) | 28.10 | 32.17 | 37.18 | 40.58 | 19.64 |
| | Main peak (%) | 59.76 | 50.85 | 44.82 | 40.12 | |
| | Basic peak (%) | 12.14 | 16.98 | 18.00 | 19.30 | |

As can be seen from Table 12, the IEC main peak contents of formulations 14-18 decreased less than that of the control formulation 12.

### 4. Screening results under freeze-thaw

The step of freeze-thaw was to freeze each sample solution at -60 °C for 24 h and then place the sample solution at 25 °C for 24 h as one freeze-thaw. The samples were detected after 0, 3, and 5 freeze-thaw cycles, and the results were as follows:

**Table 13: SEC-HPLC data under repeated freeze-thaw conditions**

| Sample | | DR0(-60°C) | DR3(-60°C) | DR5(-60°C) | DR0-DR5 (Monomer decrease %) |
|---|---|---|---|---|---|
| Formulation 6 | Aggregate (%) | 2.27 | 1.58 | 2.33 | 0.06 |
| | Monomer (%) | 96.91 | 97.63 | 96.85 | |
| | Fragment (%) | 0.81 | 0.79 | 0.83 | |
| Formulation 12 | Aggregate (%) | 2.59 | 2.70 | 2.74 | 0.19 |
| | Monomer (%) | 96.61 | 96.47 | 96.42 | |
| | Fragment (%) | 0.81 | 0.84 | 0.84 | |
| Formulation 14 | Aggregate (%) | 2.26 | 2.34 | 2.33 | 0.10 |
| | Monomer (%) | 96.92 | 96.81 | 96.82 | |
| | Fragment (%) | 0.82 | 0.85 | 0.85 | |
| Formulation 15 | Aggregate (%) | 2.28 | 2.38 | 2.42 | 0.11 |
| | Monomer (%) | 96.89 | 96.76 | 96.78 | |
| | Fragment (%) | 0.83 | 0.86 | 0.80 | |
| Formulation 16 | Aggregate (%) | 2.33 | 2.42 | 2.39 | 0.06 |
| | Monomer (%) | 96.84 | 96.77 | 96.78 | |
| | Fragment (%) | 0.83 | 0.82 | 0.83 | |
| Formulation 17 | Aggregate (%) | 2.38 | 2.42 | 2.39 | 0.02 |
| | Monomer (%) | 96.79 | 96.75 | 96.77 | |
| | Fragment (%) | 0.83 | 0.83 | 0.84 | |
| Formulation 18 | Aggregate (%) | 2.38 | 2.37 | 2.43 | 0.10 |
| | Monomer (%) | 96.80 | 96.79 | 96.70 | |
| | Fragment (%) | 0.82 | 0.84 | 0.87 | |

As can be seen from Table 13, the SEC monomer purity of formulation 12 had a decreasing trend after 5 repeated freeze-thaw cycles, while there was no significant difference in the freeze-thaw results of the other formulations.

According to the experimental results under high temperature, light, and freeze-thaw conditions, formulations 6 and 14-18 all had good stability relative to the control formulation 12.

## Claims

1. An anti-IL-5 antibody formulation comprising an anti-IL-5 antibody and a histidine buffer.

2. The formulation according to claim 1, wherein the antibody formulation has a pH value of 5.6-6.5.

3. The formulation according to claim 1, wherein the antibody formulation has a pH value of 5.7-6.4.

4. The formulation according to claim 1, wherein the antibody formulation has a pH value of 5.9, 6.0, or 6.1.

5. The formulation according to claim 1, wherein the histidine buffer is at a concentration of 10-30 mM.

6. The formulation according to claim 4, wherein the histidine buffer is at a concentration of 20 mM.

7. The formulation according to any one of claims 1-6, wherein the formulation comprises a stabilizer and/or a surfactant.

8. The formulation according to claim 7, wherein the stabilizer is sucrose or sorbitol.

9. The formulation according to claim 7, wherein the stabilizer is at a concentration of 35-130 mg/mL.

10. The formulation according to claim 8, wherein the sucrose is at a concentration of 75-130 mg/mL, or the sorbitol is at a concentration of 35-50 mg/mL.

11. The formulation according to claim 8, wherein the sucrose is at a concentration of 80 mg/mL or 120 mg/mL, or the sorbitol is at a concentration of 40 mg/mL.

12. The antibody formulation according to claim 7, wherein the surfactant is Tween 80 or Tween 20.

13. The formulation according to claim 12, wherein the Tween 80 is at a concentration of 0.1-0.4 mg/mL.

14. The formulation according to claim 12, wherein the Tween 80 is at a concentration of 0.2 mg/mL.

15. The formulation according to any one of claims 1-14, wherein the anti-IL-5 antibody is at a concentration of 80-120 mg/mL.

16. The formulation according to claim 15, wherein the anti-IL-5 antibody is at a concentration of 100 mg/mL.

17. The formulation according to any one of claims 1-16, wherein the formulation may further comprise a chelating agent.

18. The formulation according to claim 17, wherein the chelating agent is EDTA-2Na.

19. The formulation according to claim 17, wherein the chelating agent is at a concentration of 0.01-0.03 mg/mL.

20. The formulation according to claim 17, wherein the EDTA-2Na is at a concentration of 0.017 mg/mL.

21. An anti-IL-5 antibody formulation comprising 50-150 mg/mL anti-IL-5 antibody, 10-30 mM histidine buffer, 80-120 mg/mL sucrose, and 0.1-0.4 mg/mL Tween 80, wherein the antibody formulation has a pH value of 5.6-6.5.

22. An anti-IL-5 antibody formulation comprising 50-150 mg/mL anti-IL-5 antibody, 10-30 mM histidine buffer, 35-50 mg/mL sorbitol, and 0.1-0.4 mg/mL Tween 80, wherein the antibody formulation has a pH value of 5.6-6.5.

23. An anti-IL-5 antibody formulation comprising 50-150 mg/mL anti-IL-5 antibody, 10-30 mM histidine buffer, 80-120 mg/mL sucrose, 0.1-0.4 mg/mL Tween 80, and 0.01-0.03 mg/mL EDTA-2Na, wherein the antibody formulation has a pH value of 5.6-6.5.

24. An anti-IL-5 antibody formulation comprising 100 mg/mL anti-IL-5 antibody, 20 mM histidine buffer, 120 mg/mL sucrose, 0.2 mg/mL Tween 80, and 0.017 mg/mL EDTA-2Na, wherein the antibody formulation has a pH value of about 5.6-6.5.

25. An anti-IL-5 antibody formulation comprising 100 mg/mL anti-IL-5 antibody, 20 mM histidine buffer, 120 mg/mL sucrose, 0.2 mg/mL Tween 80, and 0.051 mM EDTA-2Na, wherein the antibody formulation has a pH value of about 5.6-6.5.

26. An anti-IL-5 antibody formulation comprising 100 mg/mL anti-IL-5 antibody, 20 mM histidine buffer, 80 mg/mL sucrose, 0.2 mg/mL Tween 80, and 0.017 mg/mL EDTA-2Na, wherein the antibody formulation has a pH value of about 5.6-6.5.

27. An anti-IL-5 antibody formulation comprising 100 mg/mL anti-IL-5 antibody, 20 mM histidine buffer, 80 mg/mL sucrose, 0.2 mg/mL Tween 80, and 0.051 mM EDTA-2Na, wherein the antibody formulation has a pH value of about 5.6-6.5.

28. An anti-IL-5 antibody formulation comprising 100 mg/mL anti-IL-5 antibody, 20 mM histidine buffer, 80 mg/mL sucrose, and 0.2 mg/mL Tween 80, wherein the antibody formulation has a pH value of about 5.6-6.5.

29. An anti-IL-5 antibody formulation comprising 100 mg/mL anti-IL-5 antibody, 20 mM histidine buffer, 40 mg/mL sorbitol, 0.2 mg/mL Tween 80, and 0.017 mg/mL EDTA-2Na, wherein the antibody formulation has a pH value of about 5.6-6.5.

30. An anti-IL-5 antibody formulation comprising 100 mg/mL anti-IL-5 antibody, 20 mM histidine buffer, 40 mg/mL sorbitol, 0.2 mg/mL Tween 80, and 0.051 mM EDTA-2Na, wherein the antibody formulation has a pH value of about 5.6-6.5.

31. An anti-IL-5 antibody formulation comprising 100 mg/mL anti-IL-5 antibody, 20 mM histidine buffer, 40 mg/mL sorbitol, and 0.2 mg/mL Tween 80, wherein the antibody formulation has a pH value of about 5.6-6.5.

32. An anti-IL-5 antibody formulation comprising 100 mg/mL anti-IL-5 antibody, 20 mM histidine buffer, 120 mg/mL sucrose, and 0.2 mg/mL Tween 80, wherein the antibody formulation has a pH value of 5.6-6.5.

33. The formulation according to any one of claims 24-32, wherein the antibody formulation has a pH value of 5.7-6.4.

34. The formulation according to any one of claims 24-32, wherein the antibody formulation has a pH value of 5.9-6.1.

35. The formulation according to any one of claims 1-34, wherein a light chain of the anti-IL-5 antibody comprises a sequence set forth in SEQ ID NO: 1, a sequence having at least 90% identity to the sequence set forth in SEQ ID NO: 1, or an amino acid sequence having one or more conservative amino acid substitutions as compared to the sequence set forth in SEQ ID NO: 1; and/or
a heavy chain of the anti-IL-5 antibody comprises a sequence set forth in SEQ ID NO: 2, a sequence having at least 90% identity to the sequence set forth in SEQ ID NO: 2, or an amino acid sequence having one or more conservative amino acid substitutions as compared to the sequence set forth in SEQ ID NO: 2.

36. The formulation according to claim 35, wherein the anti-IL-5 antibody has a light chain amino acid sequence set forth in SEQ ID NO: 1 and a heavy chain amino acid sequence set forth in SEQ ID NO: 2.

37. The formulation according to any one of claims 1-34, wherein the anti-IL-5 antibody is mepolizumab.

38. The formulation according to any one of claims 1-36, wherein the formulation is stable for at least 2 weeks or at least 4 weeks when stored at 40 °C; or the formulation is stable when stored under a light condition for at least 14 days; or the formulation is stable after at least 5 repeated freeze-thaw cycles.

39. A method for preparing the formulation according to any one of claims 1-38, comprising:
taking ingredients according to the formulation, adding water for dissolving and mixing the ingredients uniformly, and adjusting the pH value to 5.6-6.5 to obtain the antibody formulation;
or comprising: preparing a histidine buffer, exchanging the anti-IL-5 antibody into the histidine buffer by ultrafiltration, adding an excipient, and diluting the antibody to a specified concentration to obtain the antibody formulation.

40. Use of the formulation according to any one of claims 1-38 in the preparation of a product for treating a disease selected from the group consisting of asthma, severe eosinophilic asthma, severe asthma, uncontrolled eosinophilic asthma, eosinophilic asthma, sub-eosinophilic asthma, chronic obstructive pulmonary disease, eosinophilic granulomatosis with polyangiitis, hypereosinophilic syndrome, nasal polyposis, bullous pemphigoid and eosinophilic esophagitis.

41. An antibody pharmaceutical product for treating an IL-5 related disease comprising the formulation according to any one of claims 1-38 and a container for storing the formulation.
